# EUROPÄISCHE PATENTANMELDUNG

(11) **EP 1 669 020 A1**
(43) Veröffentlichungstag der Anmeldung: **14.06.2006**
(21) Anmeldenummer: 04028895.3
(22) Anmeldetag: 07.12.2004
(51) Int. Cl.: A61B 5/00

(54) **Aufbewahrungstasche mit integrierten Funktionen**

(71) Anmelder: Roche Diagnostics GmbH, 68305 Mannheim (DE); F. HOFFMANN-LA ROCHE AG, 4070 Basel (CH)
(72) Erfinder: Rasch-Menges, Jürgen, 68723 Schwetzingen (DE); Jansen, Paul, 68163 Mannheim (DE)
(74) Vertreter: Poredda, Andreas

(57) **Zusammenfassung**

Die Erfindung betrifft ein System zur Bestimmung der Konzentration eines Analyten in einer Körperflüssigkeit, das ein Instrument, mindestens ein analytisches Testelement sowie eine Aufbewahrungstasche, in der das Instrument und das Testelement aufbewahrt werden, beinhaltet, wobei ein Einschaltmodul und/oder ein Modul zur Inventarkontrolle in der Aufbewahrungstasche integriert sind. Das Instrument weist ein Auswertemodul zur Analyse von Daten für die Konzentrationsbestimmung auf und das analytische Testelement weist eine Nachweiszone mit Nachweischemie zum Nachweis des Analyten in einer Körperflüssigkeit auf.

## Beschreibung

Die vorliegende Erfindung betrifft Systeme zur Bestimmung der Konzentration eines Analyten aus einer Körperflüssigkeit, die eine Aufbewahrungstasche beinhalten, in der Komponenten des Systems aufbewahrt werden.

Medizintechnische Instrumente, insbesondere kompakte Geräte für den mobilen Einsatz in Krankenhaus und Rettungswagen, aber auch im privaten Bereich, zur Diagnose und Therapie werden häufig in einer Aufbewahrungstasche ausgeliefert, in der das Instrument sowie für die dazugehörigen Komponenten verstaut werden.

Insbesondere im so genannten home-use Bereich, also dort, wo die medizinischen Laien selbstständig eine einfache Analyse und gegebenenfalls eine Medikation durchführen und dort insbesondere für die regelmäßige, mehrmals durchzuführende Blutgewinnung und Insulinverabreichung durch Diabetiker für die Einstellung der Blutglukosekonzentration, werden Lanzetten und dazu passende Geräte, so genannte Stechhilfen angeboten, die eine möglichst schmerzarme und verlässliche Blutgewinnung ermöglichen. Als Beispiel für Lanzetten und Stechhilfen seien die kommerziell erhältlichen Geräte (Stechhilfen und Lanzetten) Glucolett der Bayer AG und Softclix® der Roche Diagnostics GmbH genannt. Solche Lanzetten und Geräte sind z. B. in WO 98/48695, EP 0 565 970, US 4,442,836 oder US 5,554,166 beschrieben. Blutzuckermessgeräte im Stand der Technik, wie z. B. AccuChek Sensor® (von Roche Diagnostics GmbH) bestehen aus einem Messgerät, in das ein Testelement (Teststreifen) eingeführt wird. Der Teststreifen wird mit einem Bluttropfen in Kontakt gebracht, der zuvor mittels der Stechhilfe aus der Fingerbere gewonnen wurde. Das Blut wird auf dem Teststreifen in den Bereich transportiert, in dem sich die Nachweischemie befindet. Dort reagiert das gesuchte Analyt mit der Nachweischemie und generiert dabei ein messbares Signal, z. B. einen elektrischen Stromimpuls oder eine Farbänderung. Das Messsignal wird vom Messgerät ausgewertet und auf dem Display des Blutzuckermessgeräts wird dem Benutzer der aktuelle Blutzuckerwert angezeigt.

Die Verabreichung des Insulins geschieht häufig mit so genannten Insulinpens, beispielsweise dem Insulinpen OptiPen® der Firma Aventis, bei dem eine fest eingestellte Menge Insulin als Bolusinjektion in das Unterhautfettgewebe gesprizt wird.

Für eine kontinuierliche Insulinabgabe können Insulinpumpen verwendet werden, z. B. die H-Tron plus® und D-Tron plus® von Disetronic. Die Pumpe wird üblicherweise auf dem Bauch befestigt und über einen transkutanen Katheter wird der Anwender mit Insulin versorgt.

### Stand der Technik

Bei heutzutage auf dem Markt erhältlichen diagnostischen Geräten für den home-use Gebrauch, z. B. Accu-Chek Comfort®, Accu-Chek Active®, und Accu-Chek Compact® von Roche Diagnostics GmbH oder auch das Lifescan OneTouch Ultra® von Lifescan, sowie Bayer Glucometer Dex® der Firma Bayer AG, wird dem Kunden eine Aufbewahrungstasche mitgeliefert. Diese Aufbewahrungstasche besteht meist aus einem mit einem Reissverschluss versehenen mittig aufklappbaren Mäppchen oder einer einfachen Einschubhülle. In dem Mäppchen befinden sich zumeist Gummibänder oder Schlaufen, welche die Komponenten fixieren, sowie einige Fächer zur Aufbewahrung von Hilfmitteln wie Batterien. Die Aufbewahrungstasche ist meist aus einem weichen zuweilen gepolsterten, oder thermo isolierten Material, zumeist grau-schwarzes Nylon. Bei einigen Systemen ist die Tasche aus einem harten Kunststoff, wie z. B. das Hard-Case für das Accu-Chek Go® von Roche Diagnostics GmbH. Außer der Aufbewarungs- und Staufunktion sind jedoch keine weiteren Funktionen in die Aufbewahrungstasche integriert. Als Verschluss der Tasche dienen meist Reißverschlüsse, Druckknöpfe, Klettverschlüsse oder Ähnliches.

EP 1 079 876 beschreibt ein Blutglukosesystem, wobei das Gehäuse des Blutglukosemeters so geformt ist, dass verschieden Komponenten (Lanzetten, Testträgervorrat, Insulinpen) im Gehäuse eingelegt werden können und die Stechhilfe am Gehäuse befestigt werden kann. Das so zusammengesetzte System wird anschließend in eine Aufbewahrungstasche gesteckt.

Im Gehäuse des InDuo® Glukosemessgeräts von Lifescan ist ein Einschub vorgesehen, in den der Insulinvorrat und die Insulinspritze eingeschoben werden können. Auf diese Weise nimmt das System wenig Platz weg und das Insulin ist durch das Hartkunststoff Gehäuse des Messgeräts mechanisch geschützt.

Bei dem GlukoMON® System der Firma Diabetech kann das Blutglukosemessgerät nach Abschluss der Blutglukosemessung wieder in die Aufbewahrungstasche zurück gestellt werden und dort über einen seriellen Anschluss an einen Sender, der sich in der Aufbewahrungstasche befindet, angeschlossen werden. Der Sender funkt beispielsweise einen Pager an, mit dem z. B. die Eltern eines Diabetiker-Kindes zu informieren, wann ihr Kind seinen Blutzucker gemessen hat und welchen Blutzuckerwert es dabei hatte. D. h. der in der Aufbewahrungstasche integrierte Sender erfüllt eine reine Informations-Funktion und hat mit der eigentlichen Messung nichts zu tun.

Weitere Aufbewahrungsbehältnisse im Stand der Technik sind beispielsweise in US Des. 337,658 und US D439,669 beschrieben. Hierbei handelt es sich um Aufbewahrungstaschen aus Hartkunststoff, die der Aufbewahrung und dem mechanischen Schutz dienen.

Im Zuge der Entwicklung hin zur Integration von immer mehr Funktionen im Instrument ist es schwierig den gleichzeitigen Wunsch nach immer kleinerer Baugröße des Instruments nachzukommen. Darüberhinaus müssen meist nicht nur bei einer kompletten Neuentwickling, sondern auch bei einer einfachen Weiterentwicklung innerhalb einer Plattform, fast alle Module des Systems angepasst werden z. B. auf Grund eines neuen Designs des Gehäuses.

### Aufgabe

Aufgabe der Erfindung ist es, ein System zur Bestimmung der Konzentration eines Analyten in einer Körperflüssigkeit bereitzustellen, das eine Aufbewahrungstasche beinhaltet, in der Komponenten des Systems verstaut werden können, wobei die Aufbewahrungstasche eine gegenüber dem Stand der Technik verbesserte Funktionalität aufweist.

### Beschreibung der Erfindung

Eine erste Ausführungsform der Erfindung betrifft ein System zur Bestimmung der Konzentration eines Analyten in einer Körperflüssigkeit, das ein Instrument, analytische Testelemente sowie eine Aufbewahrungstasche, in der das Instrument und das Testelement aufbewahrt werden, beinhaltet, wobei ein Einschaltmodul und / oder ein Modul zur Inventarkontrolle in der Aufbewahrungstasche integriert sind. Das Instrument weist ein Auswertemodul zur Analyse von Daten für die Konzentrationsbestimmung auf und das analytische Testelement weist eine Nachweiszone mit Nachweischemie zum Nachweis des Analyten in einer Körperflüssigkeit auf.

Das erfindungsgemäße System umfasst die Komponenten, die zur Bestimmung der Konzentration eines Analyten in einer Körperflüssigkeit notwendig sind. Minimal beinhaltet das System ein Instrument mit einem Auswertemodul, ein analytisches Testelement mit einer Nachweiszone sowie eine Aufbewahrungstasche für Instrument und Testelement. Insbesondere kann das System darüber hinaus beispielsweise eine Stechhilfe mit einer Lanzette, eine Anzeige- und eine Bedieneinheit sowie gegebenenfalls auch einen Insulinpen umfassen, wobei diese Komponenten sowohl in Testelement bzw. Instrument integriert als auch als eigenständige Module realisiert sein können.

Beispielsweise beinhaltet ein System zur Blutglukosemessung typischer Weise ein Instrument, eine Stechhilfe, ein Vorratsbehälter mit mehreren Teststreifen, einen Vorratsbehälter mit mehreren Lanzetten und eine Aufbewahrungstasche, in der die genannten Komponenten verstaut werden können. Ein System zur Insulintherapie beinhaltet typischer Weise darüber hinaus einen Vorratsbehälter mit Insulin, z. B. in Form einer Ampulle, sowie ein Gerät zur Verabreichung des Insulins, beispielsweise eine Insulinpumpe bzw. ein Insulinpen.

Die erfindungsgemäße Lösung beinhaltet eine Auslagerung von Funktionen aus dem medizinischen Instrument in die Aufbewahrungstasche, wobei vorzugsweise die Grundfunktion und das Grunddesign der Aufbewahrungstasche erhalten bleiben. Die bestehenden Aufbewahrungstaschen werden um die ausgelagerten Zusatzfunktionen erweitert. Auslagerbare Funktionen sind beispielsweise Funktionen für die Eingabe und Anzeige von Werten oder Befehlen, die Schnittstellen zwischen Systemkomponenten sowie die Energieversorgung und insbesondere Funktionen für das Einschalten des medizinischen Instruments und die Inventarkontrolle der Systemkomponenten. Die Aufbewahrungstasche kann wie bisher aus Stoff, Kunstfaser, Leder, Kunstleder oder sonstigen Materialien hergestellt werden. Sie kann mit Reissverschlüssen, Druckverschlüssen, Clips, Druckknöpfen, Gürteln oder Schlössern verschlossen werden. Die Aufbewahrungstasche kann zum Schließen ein- oder mehrfach gefaltet, geklappt oder zusammengerollt werden. Die darin zu verstauenden Systemkomponenten können über Gummibänder, Schlaufen, Klettverschlüsse, Reissverschlüsse, Druckknöpfe oder Führungsschienen befestigt werden.

Die erfindungsgemäße Auslagerung von Modulen in die Aufbewahrungstasche ermöglicht kleinere Bauformen der Systemkomponenten, insbesondere des Auswerteinstruments. Dies ist besonders bei einem mobilen Einsatz des Systems für den Anwender von großem Nutzen. Darüber hinaus kann das System einfach um zusätzliche Funktionen erweitert werden, z. B. indem die neuen Module in die Aufbewahrungstasche eingesetzt werden bzw. indem die Tasche gegen eine andere mit der gewünschten Funktionalität ausgetauscht wird. Die übrigen Systemkomponenten, insbesondere das medizinische Instrument, brauchen nicht geändert zu werden. Die Aufbewahrungstasche bietet meist mehr Platz, so dass die Module leichter dort integriert werden können als in dem Instrument, das möglichst klein und leicht sein soll. Die Aufbewahrungstasche ist auch meist preiswerter in der Herstellung und deshalb leichter austauschbar als ein hochwertiges Instrument, das eventuell auch bereits kundenspezifisch konfiguriert wurde, was bei einem Gerätetausch wiederholt werden müsste. Darüber hinaus kann die erfindungsgemäße Auslagerung der Module die Weiterentwicklung der Systeme vereinfachen. Beispielsweise brauchen bei einer Weiterentwicklung des Instruments nur die Schnittstellen zu den Modulen, die in die Aufbewahrungstasche integriert sind, berücksichtigt zu werden; die übrigen Module selbst brauchen nicht angepasst zu werden. Auf diese Weise ist es auch möglich, die ausgelagerten Module für mehrere Geräteversionen oder -plattformen zu nutzen. Die Geräteentwicklung braucht lediglich die Schnittstellen zu den ausgelagerten Funktionen zu berücksichtigen.

Mit Analyt ist ein Bestandteil der Körperflüssigkeit gemeint, der im Nachweisbereich mit der Nachweischemie reagiert, so dass - ab einer gewissen Menge des Analyts - die Reaktion in einer Messanordnung gemessen werden kann. Eine bevorzugte Ausführungsform besteht darin, Blut als Probenflüssigkeit zu verwenden und Blutglukose als Analyt im Nachweisbereich nachzuweisen und daraus die Konzentration der Blutglukose zu ermitteln.

Neben Blut sind auch interstitielle Flüssigkeiten, Urin und andere körpereigene Flüssigkeiten als Körperflüssigkeiten möglich. Ebenfalls ist es möglich nicht nur einen Analyten, z. B. Blutglukose, sondern auch mehrere Analyten nachzuweisen und dies sowohl aus einer Körperflüssigkeit, z. B. Blut, als auch aus einem Gemisch mehrerer Körperflüssigkeiten, z. B. Blut und interstitielle Flüssigkeit, durchzuführen.

Eine weitere bevorzugte Ausführungsform besteht darin, Koagulationsparameter, insbesondere ProBNT, in einer Blutprobe zu analysieren. Eine dritte bevorzugte Ausführungsform besteht darin, Urinproben zu analysieren, beispielsweise hinsichtlich ihrer Konzentration an Kreatinin und Harnstoff.

Unter einem analytischen Testelement ist jede Form von trägergebundenen Schnelltest für die Diagnostik zu verstehen, insbesondere Schnelltests in Streifenform, so genannte Teststreifen, hierbei insbesondere zur Bestimmung des Blutglukosegehalts bei Diabetikern. Darüber hinaus können Teststreifen zur Diagnose von Koagulationsparametern und von Urinproben verwendet werden.

Das medizinische Instrument bezeichnet einen Teil des Systems, der Veränderungen, die im Nachweisbereich des analytischen Testelements erzeugt werden, detektiert und in einem Auswertemodul auswertet. Bei foto-optischen Systemen wird hierbei beispielsweise ein Farbumschlag gemessen, bei elektro-chemischen Systemen beispielsweise eine Stromsignal. Das Instrument kann zusätzlich eine Halterung enthalten, in der ein analytisches Testelement oder eine Verpackung für ein oder mehrere Testelemente, z. B. ein Container oder ein Magazin, positioniert wird. Darüber hinaus kann auch eine Anzeigeeinheit, z. B. ein LCD-Display, in das Instrument integriert sein. Bevorzugter Weise stellt das Instrument ein batteriebetriebenes Handgerät da.

Mit Aufbewahrungstasche ist jede Art von Behältnis zu verstehen, in dem das Instrument und ggf. weitere Systemkomponenten aufbewahrt werden können. Beispielsweise handelt es sich bei den Aufbewahrungstaschen um weiche Mäppchen, die gefüttert oder thermo-isoliert sein können und beispielsweise aus robustem Kunststoff, wie Kunstleder oder Nylongewebe, hergestellt sind. Darüber hinaus ist es jedoch auch möglich, dass die Aufbewahrungstasche aus einer harten Schale besteht, beispielsweise aus Hartkunststoff oder auch Metall. Beispielsweise besteht die Aufbewahrungstasche aus zwei Hartkunststoff-Halbschalen, die über ein Gelenk verbunden und so geöffnet werden können, oder die Aufbewahrungstasche besteht aus einer Art Behälter mit einem Deckel. Die Aufbewahrungstasche kann auch offene oder verschließbare Einschübe aufweisen. Je nach Ausführungsform können in der Aufbewahrungstasche ein Teil oder auch alle Komponenten des Systems verstaut werden. Hierzu befinden sich in der Aufbewahrungstasche zumeist Gummibänder, Gummischlaufen oder Einschubfächer, mit deren Hilfe die Komponenten fixiert werden können. Sie schützt die Komponenten vor Verschmutzung und Beschädigung und kann darüber hinaus der Aufbewahrung weiterer Utensilien dienen.

Das Einschaltmodul beinhaltet die Funktion des Einschaltens des Instruments. Beispielsweise kann dies durch einen separaten Einschaltknopf oder -taste realisiert sein. Oder das Gerät kann durch Aufklappen einer Klappe, die z. B. Display und Bedientasten schützt, eingeschaltet werden. Andere Geräte, wie beispielsweise das AccuChek Active® von Roche Diagnostics schalten sich automatisch ein, sobald ein Teststreifen in das Gerät eingeschoben wird. Darüber hinaus ist es auch möglich, dass das Einschaltmodul so realisiert ist, dass durch Drücken einer beliebigen Taste das Instrument eingeschaltet wird. Da es sich bei den erfindungsgemäßen Instrumenten typischerweise um batteriebetriebene Geräte handelt, ist es sinnvoll, dass sich die Geräte, wenn sie nicht in Gebrauch sind, abschalten um keinen elektrischen Strom zu verbrauchen. Daher müssen sie für eine Konzentrationsbestimmung jedes Mal eingeschaltet werden. Das Eingabemodul kann ebenfalls beispielsweise durch Tasten oder Knöpfe realisiert sein. So weisen die Blutglukosemessgeräte typischerweise 1 - 3 Eingabetasten auf. Darüber hinaus ist es auch möglich, dass die Eingabefunktion in dem Anzeigemodul, beispielsweise durch einen Touchpad Display, integriert ist. Das Energieversorgungsmodul besteht typischerweise aus ein oder mehreren Batterien oder Akkus.

Darüber hinaus kann das Energieversorgungsmodul beispielsweise eine Akkuladestation bzw. ein Netzteil mit einem Anschluss an eine Steckdose beinhalten.

Die Integration von Einschaltmodul und/oder Modul zur Inventarkontrolle in der Aufbewahrungstasche ist erfindungsgemäß so zu verstehen, dass die Module entweder fest in der Aufbewahrungstasche installiert sind oder dass die Module aus der Aufbewahrungstasche entnehmbar sind. Mit integrierten Modulen sind hierbei Module gemeint, die sich direkt in der Aufbewahrungstasche befinden, beispielsweise im Futter oder in einer Halbschale der Aufbewahrungstasche, und nicht Module, die sich in anderen Komponenten des Systems befinden und diese Komponenten werden in der Aufbewahrungstasche aufbewahrt. Insbesondere ist mit in der Aufbewahrungstasche integrierten Modulen nicht gemeint, dass sich beispielsweise das Einschaltmodul und/oder das Inventarmodul in dem medizinischen Instrument für die Auswertung befinden und das medizinische Instrument in der Aufbewahrungstasche verstaut ist. Erfindungsgemäß werden integrierte Module, insbesondere auch entnehmbare Module, im Normalfall benutzt während sie sich in der Aufbewahrungstasche befinden.

Entnehmbare Module können in Fächer oder Taschen innerhalb der Aufbewahrungstasche hineingesteckt werden und/oder durch Gurte, Klettverschluss, Reißverschluss, Druckknöpfe oder Ähnliches fixiert werden. Die Module können leicht vom Benutzer entnehmbar sein, beispielsweise ein eingeschobenes Netzteil, das für einen mobilen Einsatz einfach aus der Aufbewahrungstasche entfernt werden kann, oder die Module, oder ein Teil der Module sind nur von autorisiertem Personal zu entnehmen.

Fest installierte Module sind nicht zur Entnahme vorgesehen, sie sind beispielsweise eingenäht bzw. an die Aufbewahrungstasche angeklebt bzw. in sie eingeklebt. Sie können auch in die Aufbewahrungstasche eingegossen oder auf eine andere Weise dauerhaft befestigt sein.

Der gesuchte Analyt, z. B. die Blutglukose, reagiert im Nachweismodul auf dem analytischen Testelement mit der Nachweischemie und erzeugt dabei ein messbares Signal, z. B. einen elektrischen Stromimpuls oder eine Farbänderung. Das Messsignal wird auf dem analytischen Teststreifen oder im Instrument gemessen und im Auswertemodul ausgewertet. Das Anzeigemodul zeigt dem Benutzer schließlich den ermittelten Wert, beispielsweise die Konzentration der Blutglukose im Blut, an. Als Anzeigemodul dient typischerweise ein gut lesbares LCD Display. Üblicherweise ist das Anzeigemodul in das Instrument integriert, es ist jedoch auch möglich, dass das Modul in einer eigenständigen Einheit realisiert ist. Beispielsweise kann eine Art Armbanduhr, die am Handgelenk befestigt wird, oder aber auch das Display eines Handys oder eines PDA's als Anzeigemodul dienen.

Eine bevorzugte Ausführungsform der Erfindung betrifft ein System zur Bestimmung der Konzentration eines Analyten in einer Körperflüssigkeit, das ein Instrument mit Auswertemodul zur Auswertung von Daten, ein analytisches Testelement mit einer Nachweiszone mit Nachweischemie zum Nachweis des Analyten in einer Körperflüssigkeit, eine Lanzette zum Erzeugen einer Körperöffnung, eine Stechhilfe, die die Bewegung der Lanzette bei der Erzeugung der Körperöffnung steuert, sowie eine Aufbewahrungstasche, in der die genannten Komponenten aufbewahrt werden, beinhaltet, wobei ein Einschaltmodul und / oder ein Modul zur Inventarkontrolle in der Aufbewahrungstasche integriert sind.

Eine Lanzette umschreibt ein zylindrisches oder flaches Bauteil, beispielsweise aus Metall, Kunststoff, Silizium, vorzugsweise aus rostfreiem Edelstahl, mit dem möglichst schmerzfrei eine Körperöffnung erzeugt werden kann, beispielsweise an der Fingerbeere oder am Unterarm, um einen kleinen Tropfen Blut für die Bestimmung der Konzentration eines Analyten erzeugen zu können, z. B. Blutglukose oder ein Koagulationsparameter, beispielsweise ProBNT. Zu diesem Zweck ist die Lanzette meist spitz zugeschnitten und weist einen speziellen Schliff auf. Eine Stechhilfe steuert die Bewegung der Lanzette. Durch eine geschickte Bewegungsführung der Lanzette kann das Schmerzempfinden beim Erzeugen der Körperöffnung deutlich reduziert werden. Ein Beispiel für eine Stechhilfe ist der Softclix® der Firma Roche Diagnostics, der ähnlich einem Kugelschreiber ausgeformt ist. Die Spitze der Lanzetten weist üblicherweise einen Sterilschutz auf, der gewährleistet, dass der Teil der Lanzette, der in den Körper eindringt, bis zum Gebrauch steril bleibt, um Kontaminationen zu vermeiden. Bei heute üblichen Systemen werden meist die Lanzetten lose in einem Vorratsbehälter geliefert und manuell vom Benutzer einzeln in die Stechhilfe eingelegt. Der Spitzenschutz wird entfernt, die Stechhilfe wird an die zu stechende Stelle, z. B. die Fingerbeere, angepresst und ausgelöst. Die Lanzette taucht schnell und kurz in den Finger ein und zieht sich wieder zurück, und erzeugt so eine punktförmige Wunde, aus der ein Tropfen als Blutprobe gewonnen und auf das Testelement aufgebracht werden kann. Es ist aber auch möglich, dass die Stechhilfe bereits mit einer Lanzette geladen ist oder sogar einen Lanzettenvorrat beinhaltet. Nach Gebrauch kann die benutzte Lanzette verworfen werden und die nächste Lanzette wird automatisch oder durch eine manuell bedienbare Mechanik aus dem Vorrat in die Stechposition der Stechhilfe transportiert. Ein solches System eines automatischen Lanzettenapparates ist beispielsweise in US 5,152,775 beschrieben.

Darüber hinaus ist es auch möglich, dass die Lanzette in dem analytischen Testelement integriert ist. Vorzugsweise ist bei einem solchen so genannten integrierten Disposable auch die Stechhilfe in das Instrument integriert.

In einer weiteren Ausführungsform befinden sich ein oder mehrere analytische Testelemente bzw. ein oder mehrere integrierte Disposables in dem Messinstrument und das Instrument wird in der Aufbewahrungstasche verstaut.

Eine weitere Ausführungsform der Erfindung betrifft ein erfindungsgemäßes System, wobei das System zusätzlich der Verabreichung eines Medikaments dient und ein Instrument aufweist, das ein Medikationsmodul zur Verabreichung eines Medikaments beinhaltet. Dieses Instrument kann beispielsweise ein Insulinpen oder eine Insulinpumpe sein. Das Insulin kann z. B. in einer Glas- oder Kunststoffampulle vorliegen und vor jeder Applikation in eine Spritze aufgezogen und beispielsweise in die Bauchdecke injiziert werden, oder es liegt bereits in einer Spritzenampulle bzw. in einem Insulinpen vor. Bei einer Insulinpumpe ist es darüber hinaus möglich, kontinuierlich in kleinen Dosen eine so genannte Basalrate an Insulin abzugeben und zusätzlich können durch manuelles Auslösen Bolusinjektionen gegeben werden. Bei der Insulintherapie kann der Benutzer beispielsweise zuerst seinen Blutzuckerwert messen, indem er zunächst mit Lanzette und Stechhilfe eine Blutprobe gewinnt, sie auf das analytische Testelement aufbringt, im Blutzuckermessgerät die Konzentration der Blutglukose im Blut bestimmt und daraus beispielsweise über Tabellen ermittelt, ob und wie viel Insulin er sich zu injizieren hat um seinen Blutzuckerwert im gewünschten Bereich zu halten. Darüber hinaus ist es natürlich auch möglich, dass andere Medikamente verabreicht werden. Sowohl die Bestimmung der Konzentration als auch die Verabreichung eines Medikaments kann einerseits von dem Laien selbst, oder aber auch von einem Professionellen, beispielsweise einem Arzt oder einer Krankenschwester, z. B. bei der Benutzung in einem Krankenhaus, durchgeführt werden. Ein solches System besteht beispielsweise aus einem Instrument zur Bestimmung der Konzentration, einem Vorratsbehälter mit Teststreifen, einem Insulinpen und einer Aufbewahrungstasche, in der die Komponenten untergebracht sind.

In einer weiteren Ausführungsform sind in der Aufbewahrungstasche das Messinstrument sowie die Teststreifen, z. B. in einem Vorratsbehälter, untergebracht, und eine Insulinpumpe ist auf der Bauchdecke fixiert, die über einen transkutanen Katheder kontinuierlich Insulin in den Körper abgibt und darüber hinaus über ein Bedienelement, das sich vorzugsweise an der Pumpe befindet, können zusätzliche Insulinboli verabreicht werden.

Das Instrument mit dem Auswertemodul und das analytische Testelement können separat vorliegen, es ist darüber hinaus aber auch möglich, dass sich ein oder insbesondere mehrere Testelemente in dem Instrument befinden und dass das Instrument in einer entsprechenden Aufbewahrungstasche aufbewahrt wird.

Eine weitere Ausführungsform der Erfindung betrifft ein erfindungsgemäßes System, das Einschaltmodul, ein Eingabemodul, ein Nachweismodul, ein Auswertemodul, ein Anzeigemodul, ein Energieversorgungsmodul und ein Stechmodul beinhaltet. Ein solches System ist insbesondere geeignet für die Bestimmung von Blutglukose, Koagulationsparametern und für die Durchführung einer Insulintherapie. Insbesondere kann mit einem solchen System der folgende Prozess durchgeführt werden: Erzeugen einer Körperöffnung durch Stechen mit einer Stechhilfe mit Lanzette, Nachweis eines Analyten, z. B. Blutglukose, mit einem analytischen Testelement, Auswerten der Messung in einem Instrument, und Anzeige der Konzentration des Analyten in einer Körperflüssigkeit. Das Stechmodul beschreibt eine Einheit, mit der eine Körperöffnung erzeugt werden kann, insbesondere kann hierzu eine Lanzette zusammen mit einer Stechhilfe genutzt werden.

Eine weitere Ausführungsform betrifft ein System wie eben beschrieben, dass zusätzlich ein Medikationsmodul beinhaltet. Ein solches System kann insbesondere zur Insulintherapie dienen wobei zunächst die Blutglukose bestimmt wird und anschließend entsprechend Insulin injiziert wird. Das Medikationsmodul dient der Verabreichung eines Medikaments und kann einerseits eine herkömmliche Spritze sein, es ist aber insbesondere zur Verabreichung von Insulin möglich, wobei es sich dabei um einen Insulinpen oder eine Insulinpumpe handeln kann.

Die Kommunikation zwischen den in der Aufbewahrungstasche integrierten Modulen und anderen Modulen des Systems kann beispielsweise über elektrische Kontakte erfolgen. Die Kontakte können über Steckverbindungen wie beispielsweise Klinkenstecker oder andere Stecker, insbesondere solche, die üblicherweise bei Computern Verwendung finden, realisiert werden. Darüber hinaus kann der elektrische Kontakt auch durch Auflagekontakte oder Mikroschalter hergestellt werden. Es ist auch möglich, dass die Kommunikation zwischen den Modulen durch drahtlose Kommunikationsmittel erfolgt.

Die Kommunikation zwischen den Modulen des Systems kann beispielsweise von einem gängigen Busprotokoll übernommen werden, von einfachen Schaltern in einem Stromkreis über serielle oder parallele Protokolle bis hin zu CAN-, Bus- oder Netzwerkprotokollen. Elektrische Leiterbahnen können in oder auf der Aufbewahrungstasche bzw. im Material der Aufbewahrungstasche oder zwischen verschiedenen Lagen des Materials verlegt werden. Es können gängige Niederstromkabel beispielsweise PC-Kabel, Folien, Platinen, Flachbandkabel oder flexible Leiterbahnen zum Einsatz kommen. Insbesondere ist es möglich, dass die Leiterbahnen oder auch weitere Bauteile der Elektronik in der Aufbewahrungstasche durch Polymerelektronik realisiert werden. Elektrische Kontakte können beispielsweise durch serielle, parallele oder USB-Stecker LAN, RJ45, Firewire u. s. w. realisiert werden. Drahtlose Verbindungen können durch Infrarot-Schnittstellen, Ultraschallsensoren, Bluetooth, W-LAN, die in oder an der Aufbewahrungstasche integriert sind, realisiert werden. Insbesondere können Radiofrequenzübertragungen (z. B. Transpondersysteme) für eine kontaktlose Verbindung verwendet werden. Für die Logik kann ein ASIC-Baustein oder sonstige embedded systems (z. B. CPU, RAM oder andere Schnittstellen) verwendet werden. Die Logik kann eigenständig in der Aufbewahrungstasche vorhanden sein, sich Ressourcen mit einem eingelegten Gerät teilen, oder sich auch vollständig in dem eingelegten Gerät befinden.

Eine erste Ausführungsform der Erfindung betrifft ein erfindungsgemäßes System wobei ein Einschaltmodul in der Aufbewahrungstasche integriert ist. Beispielsweise beinhaltet die Aufbewahrungstasche ein Instrument und einen Vorrat mit analytischen Testelementen und die Aufbewahrungstasche wird durch einen Reißverschluss oder ein Druckknopf verschlossen. Hierzu wird beispielsweise der Einschaltstromkreis des Geräts in die Aufbewahrungstasche geführt. Öffnet der Kunde die Aufbewahrungstasche so wird der Stromkreis geschlossen und das Gerät schaltet sich ein. Während der Kunde das Instrument bzw. das analytische Testelement oder die Stechhilfe handhabt, kann zeitgleich z. B. ein Selbsttest im Instrument durchgeführt werden, und falls das Gerät beispielsweise ein Magazin mit Teststreifen beinhaltet auch schon ein Testträger in die Probenaufnahmeposition gebracht werden. Das Instrument kann hierbei so in der Aufbewahrungstasche fixiert sein, dass eine Handhabung bzw. Nutzung ohne Entnahme möglich ist.

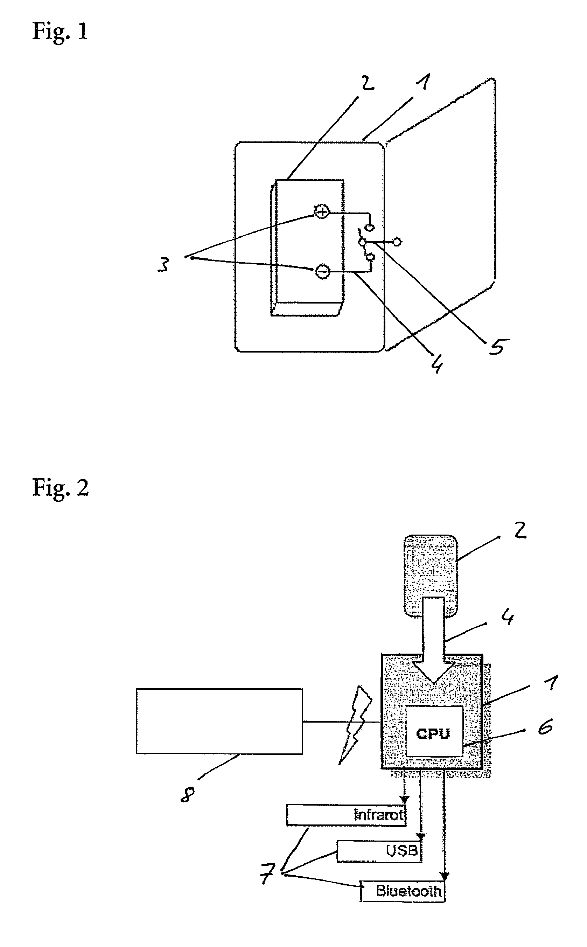

Figur 1 zeigt eine Aufbewahrungstasche (1), in der sich ein Instrument (2) befindet, dessen Ein-/ Ausschalter durch Kontakte (3) aus dem Instrument in die Aufbewahrungstasche geschleift sind. Das Instrument wird z. B. durch eine Schlaufe in der Aufbewahrungstasche fixiert und ist ansonsten ein eigenständiges Gerät. Ein Schalter (4) schließt die Schleife über einen Mechanismus (5) kurz, wenn die Aufbewahrungstasche geöffnet wird, woraufhin sich das Gerät einschaltet. Neben mechanischen Schaltern können auch kontaktlos arbeitende Sensoren, z. B. Lichtsensoren, verwendet werden. Ein Vorteil dieser Ausführungsform besteht darin, dass das Instrument bereits beispielsweise mit einer Initialisierung und einem Selbsttest beginnt, sobald die Aufbewahrungstasche geöffnet wird. Währenddessen kann der Benutzer beispielsweise einen Teststreifen aus dem Magazin holen und bereitlegen, eine Lanzette einsetzen und mit der Stechhilfe eine Blutprobe generieren, d. h. die Handhabung des Systems wird vereinfacht und beschleunigt. Gegebenenfalls gibt es zusätzlich einen Ein-/Ausschalter am Instrument, beispielsweise um ein Gerät einzuschalten, das sich nicht außerhalb der Aufbewahrungstasche befindet.

Eine weitere Ausführungsform der Erfindung betrifft ein weiteres Modul, das in der Aufbewahrungstasche enthalten sein kann. Hierbei ist ein Modul, das Schnittstellen zu ein oder mehreren Modulen des Systems beinhaltet (Schnittstellenmodul), in der Aufbewahrungstasche integriert. Die Kommunikation über diese Schnittstellen kann sowohl leitungsgebunden über elektrische Kontakte als auch drahtlos erfolgen. Die Schnittstellen können insbesondere die Kommunikation der Module des Systems sicherstellen, die in der Aufbewahrungstasche aufbewahrt werden. Darüber hinaus ist es auch möglich, dass die Schnittstellen über drahtlose Kommunikation zu Modulen des Systems eine Kommunikation herstellen, die sich außerhalb der Aufbewahrungstasche befinden, beispielsweise zu einer Insulinpumpe, einem elektronischen Diabetiker Tagebuch u. s. w. So kann beispielsweise ein in der Aufbewahrungstasche befindliches Instrument die Konzentration der Blutglukose messen und den Messwert, bzw. daraus umgerechnet ein Vorschlag für eine Bolusinjektion, an die Insulinpumpe weiterreichen und / oder den Messwert an ein elektronisches Diabetiker Tagebuch übermitteln. D. h. die Aufbewahrungstasche dient hier als eine Art Verteilerknoten für Module eines Netzwerks. Diese externen Systemmodule können insbesondere Anzeigemodule, Medikationsmodule oder Module zur Dokumentation beinhalten. Diese Auslagerung des Schnittstellen-Moduls, das auch als Konnektivitätsmodul bezeichnet wird, ermöglicht beispielsweise, dass die Baugröße des Auswerteinstruments verkleinert werden kann, da Schnittstellenanschlüsse vom Instrument in die Aufbewahrungstasche verlagert werden können. Beispielsweise wird das Auswertemodul in seiner bevorzugten Aufbewahrungsposition so fixiert, dass es über Kontakte mit den in der Aufbewahrungstasche integrierten Schnittstellen verbunden ist.

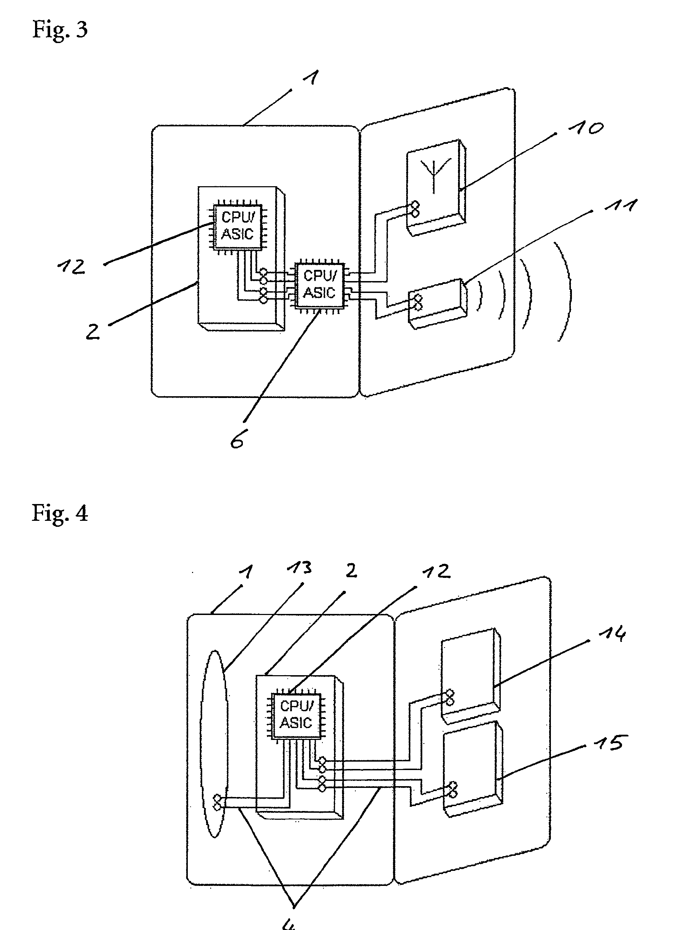

Figur 2 und 3 zeigten weitere Ausführungsformen für Schnittstellenmodule. Ein Instrument (2), ist in Figur 2 über elektrische Kontakte oder auch drahtlos mit einer CPU (6), die sich in der Aufbewahrungstasche (1) befindet, verbunden, wobei die CPU beispielsweise Infrarot, USB und/oder Bluetooth (7) ansteuern kann. Die Energieversorgung hierfür kann entweder durch eine eigene Energieversorgung in der Aufbewahrungstasche realisiert werden, beispielsweise durch einen Akku, eine Batterie oder, wie hier dargestellt, ein Netzteil (8), oder die Energie kann auch vom Instrument bereitgestellt werden.

Figur 3 zeigt eine Aufbewahrungstasche (1), in die eine CPU (6) integriert ist, sowie eine RF-Antenne (10) und eine Bluetooth- bzw. Infrarotschnittstelle (11). Diese CPU ist über Kontakte mit dem Auswerteinstrument (2) verbunden, das eine CPU (12) für die Auswertung besitzt.

Eine zweite Ausführungsform der Erfindung betrifft ein erfindungsgemäßes System, wobei ein Modul zur Inventarkontrolle (Inventarmodul) in der Aufbewahrungstasche integriert ist. In dieser Ausführungsform übernimmt die Aufbewahrungstasche die Inventarverwaltung der Systemkomponenten. Beispielsweise wird die Aufbewahrungstasche während des Verpackungsprozesses entsprechend einer Inventarliste befüllt, oder die Tasche wird leer zum Kunden geliefert und dieser befüllt die Aufbewahrungstasche entsprechend einer festen Vorgabe bzw. nach eigenen Wünschen und Bedürfnissen. Das Inventarmodul erkennt entweder selbstständig den Stand der Befüllung der einzelnen Komponenten oder es wird aktiv initialisiert, beispielsweise in der Produktion oder von dem Benutzer. Hierbei wird der Inventarzähler für die verschiedenen Komponenten, insbesondere für die Verbrauchsgegenstände wie Lanzetten und Teststreifen (Disposables), zunächst auf voll gesetzt bzw. bei individueller Befüllung auf den entsprechenden Befüllungsstand. Wird die Aufbewahrungstasche geöffnet und einer oder mehrere der bevorrateten Disposables entnommen, oder wird eine Messung mit einem Instrument durchgeführt, insbesondere wenn das Instrument ein Testelementemagazin beinhaltet, so wird die Entnahme durch Kontakte, Schalter oder Ähnliches dem Inventarmodul mitgeteilt. Die in der Aufbewahrungstasche verstauten Komponenten sind so positioniert, dass einerseits die Kontakte sicher geschlossen sind und sich die Komponenten andererseits problemlos entnehmen lassen. In einer weiteren Ausführungsform der Inventarkontrolle wird beispielsweise die Entnahme des Auswerteinstruments von der Aufbewahrungstasche erkannt und als Durchführung eines Tests interpretiert, d. h. zumindest ein Teil der zu diesem Test notwendigen Verbrauchsgegenstände, insbesondere ein analytisches Testelement, werden als gebraucht gekennzeichnet bzw. der Inventarzähler für diese Komponenten wird um eins vermindert. Liegen die Teststreifen beispielsweise in einem Container oder Magazin vor, so kann das Öffnen des Magazins beispielsweise durch elektrische Kontaktsensoren registriert und als Entnahme interpretiert werden und entsprechend der Zählerstand um eins dekrementiert werden. Analog kann beispielsweise mit einem Lanzettenmagazin verfahren werden, wobei zu beachten ist, dass die Lanzetten unter Umständen für mehrere Tests verwendet werden können.

In einer weiteren Ausführungsform beinhaltet ein Teststreifenmagazin eine Teststreifenauswurfmechanik und bei Betätigung dieser Mechanik wird die Inventarkontrolle aktiviert, sprich der Zählerstand für die Teststreifen um eins vermindert. Entsprechend kann bei einem Lanzettenmagazin mit einer entsprechenden Auswurfmechanik der Inhalt kontrolliert werden. Befindet sich ein solches Teststreifenmagazin beispielsweise in einem Auswerteinstrument, so kann auch das Instrument den Verbrauch eines Testelements registrieren und an das Inventarmodul, das sich in der Aufbewahrungstasche befindet, weitergeben, bzw. der aktualisierte Zählerstand wird über das Instrument direkt zu dem Inventarmodul in der Aufbewahrungstasche weitergegeben. Entsprechend ist es bei einem Lanzettenmagazin, das sich in oder an der Stechhilfe befindet, möglich, die Inventarkontrolle über die Stechhilfe zum Inventarmodul in der Aufbewahrungstasche weiter zu leiten. Wie oben beschrieben kann diese Weiterleitung der Information sowohl kontaktgebunden über elektrische Kontakte als auch drahtlos, beispielsweise über RF-Kommunikation, z. B. mit Transpondersystemen, vonstatten gehen.

Figur 4 - 6 beschreiben weitere Ausführungsformen für Inventarmodule. In Figur 4 übernimmt die Elektronik des Auswerteinstruments (12) die Logik für das Inventarmodul. Beispielsweise enthält die Aufbewahrungstasche (1) ein Instrument (2), das mit der Aufbewahrungstasche über Kontakte oder kontaktlos (4) kommuniziert. Das Instrument beinhaltet eine CPU bzw. einen ASIC (Application specific integrated circuit) (12), der zusätzlich zu den anderen Gerätefunktionen oder auch ausschließlich das Vorhandensein der verschiedenen Komponenten überwacht. Diese Kontakte können z. B. elektrische Schalter, Mikrotaster oder Ähnliches sein, welche die Entnahme beispielsweise der Stechhilfe (13), des Lanzettenvorrats (14) oder des Teststreifenvorrats (15) erkennen. Die Entnahme einer oder aller dieser Komponenten kann beispielsweise als die Durchführung eines Tests gewertet werden und entsprechend wird der Zählerstand um eins vermindert. Hierbei besteht das Inventarmodul aus einem Logik-Baustein und Bauteilen für die Kommunikation, z. B. elektrische Leitungen und Kontakte. Wie in Figur 4 gezeigt, sind in diesem Beispiel die Kommunikationsbauteile in der Aufbewahrungstasche integriert.

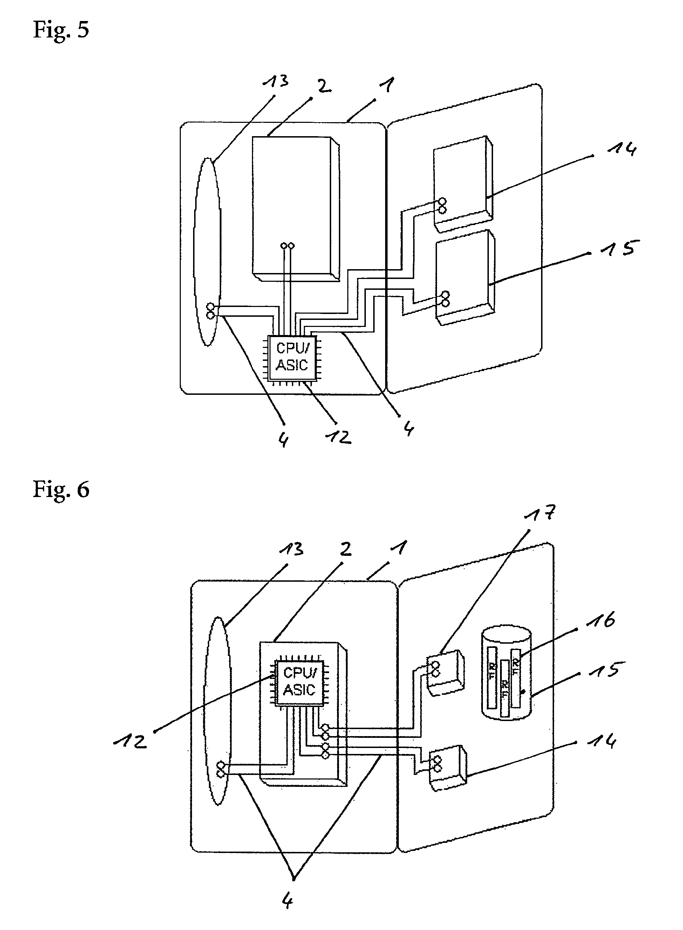

Figur 5 beschreibt ein System bei dem eine CPU bzw. ein ASIC (12) in die Aufbewahrungstasche
(1) integriert ist und über entsprechende Leitungen (4) mit den zu überwachenden Verbrauchsmaterialien verbunden ist. Auch hier kann die Entnahme von Stechhilfe (13), Lanzettenvorrat (14) und / oder Teststreifenvorrat (15) als Nutzung interpretiert werden und entsprechend der Zählerstand im Inventarmodul um eins vermindert werden. Dieser Methode liegt die Annahme zu Grunde, dass ein Anwender im Normalfall den Lanzetten- bzw. Streifenvorrat öffnet, um ein Disposable zu entnehmen und einen Test durchzuführen. Diese indirekte Art der Inventarkontrolle kann für den Anwender sehr hilfreich sein. Beispielsweise für die Planung vor längeren Abwesenheitszeiten oder als eine Art Gedächnisstütze für ältere Anwender. Beispielsweise kann so jederzeit der aktuelle Stand der Disposables abgefragt werden, ohne sie mühsam nachzählen zu müssen. Der aktuelle Stand kann automatisch immer oder zeitweise auf dem Display angezeigt werden und es ist auch möglich, einen Alarm, beispielsweise akustisch und / oder optisch zu generieren, sobald der Vorrat einer Komponente unter einen Mindestbestand sinkt.

Figur 6 beschreibt eine weitere Ausführungsform eines Inventarmoduls. Hierbei besitzt jedes einzelne analytische Testelement ein Identifikationselement, beispielsweise ein so genanntes RF-Tag (16). RF-Tags sind Transponder, mit denen kontaktlos und berührungslos Daten übertragen werden können. Auf diese Weise können in einem Magazin (15) mehrere analytische Testelemente von dem Inventarmodul registriert und gezählt werden. Hierbei beinhaltet das Inventarmodul eine entsprechende Gegenstelle, beispielsweise ein so genanntes Sendemodul (17), für das Transpondersystem. Auf diese Weise kann der aktuelle Stand an Teststreifen festgestellt und dem Benutzer angezeigt werden. Der Sende- und Empfangsbereich bei kontaktloser Datenübertragung ist räumlich soweit eingegrenzt, dass sicher gestellt ist, dass nur die Disposables bzw. Komponenten erfasst werden, die sich in der Aufbewahrungstasche befinden und nicht eventuell neben oder auf der Aufbewahrungstasche liegende weitere Komponenten erfasst werden. Beispielsweise können die entsprechenden Vorratsbehälter durch Schlaufen innerhalb der Aufbewahrungstasche fixiert werden und sind dadurch so weit positioniert, das der Sende- und Empfangsbereich räumlich stark eingegrenzt werden kann. Heutzutage können solche Identifikationselemente sehr kostengünstig beispielsweise aus Polymerelektronik hergestellt werden.

Vorzugsweise übermittelt der Transponder nicht nur die Information, dass es sich um ein analytisches Testelement handelt, sondern auch ob, es ein unbenutztes oder ein benutztes Testelement ist. Dies ist insbesondere dann von Vorteil, wenn beispielsweise ein Teststreifenmagazin in einem Auswerteinstrument integriert ist, bzw. eingelegt werden kann, und die Teststreifen nach Benutzung in dieses Magazin zurückgezogen und aufbewahrt werden, was eine hygienische Entsorgung der Teststreifen vereinfacht. Bei integrierten Disposables, bei denen Lanzette und Testelement eine Einheit bilden, wird so gleichzeitig der Stand an unbenutzten bzw. benutzten Lanzetten kontrolliert.

Darüber hinaus können mit einem RF-Tag natürlich auch weitere Informationen an das Inventarmodul übergeben werden, insbesondere Chargeninformationen und Verfallsdatum. Die Chargeninformationen können beispielsweise Auskunft darüber geben ob die einzelnen Komponenten zusammenpassen. So gehören zu jeder Charge von analytischen Testelementen ein bestimmter Satz an Kalibrationsdaten, der bei der Bestimmung der Konzentration des gesuchten Analyten berücksichtigt werden muss. Darüber hinaus kann auch das Verfallsdatum ausgewertet werden, beispielsweise kann mit einer Art Funkuhrenmodul per Funk die aktuelle lokale Zeit und Datum abgefragt werden und mit dem auf den Verbrauchsgegenständen gespeicherten Verfallsdatum, insbesondere auf den Teststreifen, verglichen und gegebenenfalls können abgelaufene Teststreifen identifiziert werden. Beinhalten die Teststreifen bzw. Teststreifencontainer beispielsweise RF-Tags, so ist es auch möglich, dass auf diesen Tags während der Produktion eine Art absolute Zeit gespeichert wird und diese Zeit mit einer entsprechenden absoluten Zeit in den anderen Komponenten, insbesondere im Inventarmodul verglichen wird. Alternativ ist auch möglich, dass in der Produktion eine Art Countdown-Mechanismus aktiviert wird, der von der Produktion bis zum Verfallstag zählt. Durch Abfrage des Zählstandes kann das Inventarmodul feststellen, ob der Teststreifen noch zu benutzen ist oder ob das Verfallsdatum schon abgelaufen ist.

Das Modul zur Inventarkontrolle kann auch auf mehrere Bauteile verteilt sein, beispielsweise ist es möglich, dass der Teststreifencontainer eine eigenständige Inventarkontrolle beinhaltet und diese Information an das Auswerteinstrument oder direkt an das in der Aufbewahrungstasche integrierte Inventarmodul weitergibt. Das Inventarmodul kann neben der Kontrolle der Verbrauchsgegenstände natürlich auch das Vorhandensein aller benötigten Komponenten des Systems überwachen beispielsweise ob eine Stechhilfe und gegebenenfalls ein Insulinpen vorhanden sind.

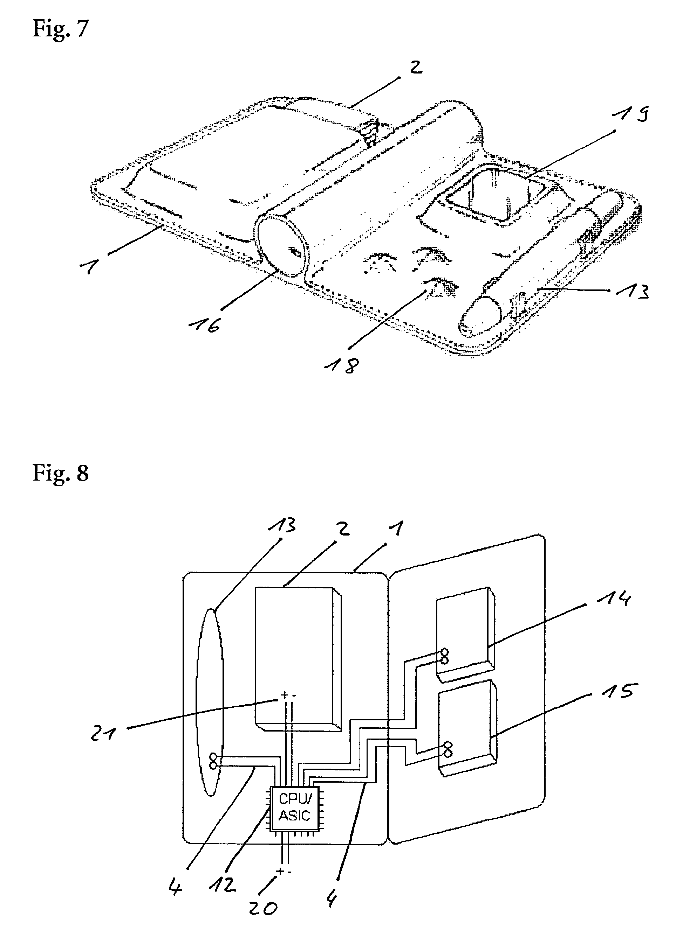

Figur 7 - 9 zeigen Beispiele für weitere Module, die zusätzlich zu Einschaltmodul und / oder Inventarmodul in der Aufbewahrungstasche enthalten sein können. In Figur 7 sind ein Eingabemodul (18) und ein Anzeigemodul (19) in der Aufbewahrungstasche (1) integriert. Von einfachen Knöpfen oder Tasten bis hin zu Tastaturen, z. B. faltbare Tastaturen, in Kombination mit einem Anzeigeelement, z. B. einem LCD-Display, können verschiedenste Bedien- und Anzeigeelemente aus dem Instrument herausgenommen und in der Aufbewahrungstasche integriert sein. Eingabe- und Anzeigemodul können beispielsweise in einem Touchscreen zu einer Einheit zusammengefasst sein. Neben dieser mechanischen Eingabe ist es auch möglich, ein Spracherkennungsmodul in die Aufbewahrungstasche zu integrieren und entsprechend kann das Anzeigemodul neben oder an Stelle einer optischen Anzeige auch eine akustische Anzeige, beispielsweise durch eine Sprachausgabe erfolgen. Auf diese Weise können auch relativ hochwertige Module verwendet werden und der Benutzer kann bei Bedarf seine Aufbewahrungstasche nachrüsten bzw. gegen eine mit mehr Funktionalität eintauschen ohne am Instrument etwas zu ändern.

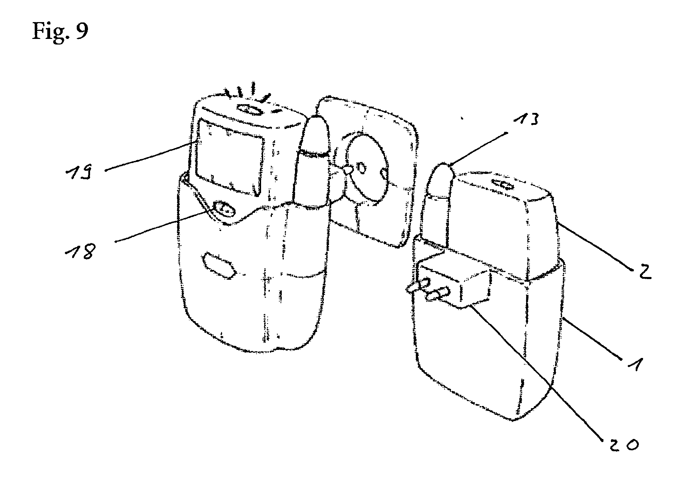

Figur 8 beschreibt ein erfindungsgemäßes System, wobei ein Energieversorgungsmodul in der Aufbewahrungstasche integriert ist (siehe Figur 8). Beispielsweise kann die Aufbewahrungstasche (1) einen Stromanschluss (20) besitzen, über welchen die Versorgungsspannung von einem externen Netzteil eingespeist wird. Üblicherweise enthält das Netzteil des Auswerteinstruments Batterien oder Akkus (21) als Energieversorgung. Diese Energiequelle kann auch für die Versorgung der in der Aufbewahrungstasche integrierten Module benutzt werden. Alternativ oder zusätzlich ist es möglich, dass die Aufbewahrungstasche Batterien und / oder Akkus beinhaltet und damit ein oder mehrere der in der Aufbewahrungstasche integrierten Module mit elektrischem Strom versorgt, beispielsweise ein elektrisches Teststreifen- oder Lanzettenmagazin oder eine elektrische Stechhilfe, ein Insulinpen oder das Auswerteinstrument. Diese Versorgung kann entweder im Routinebetrieb erfolgen oder auch als eine Art Backup für Situationen, in denen beispielsweise die Batterien oder Akkus des Auswerteinstrument leer sind. So könnte es beispielsweise möglich sein, dass das Auswerteinstrument normalerweise zur Konzentrationsbestimmung aus der Aufbewahrungstasche entnommen wird, z. B. zur leichteren Handhabung. Sind jedoch die Batterien leer, so kann das Auswerteinstrument auch in der Aufbewahrungstasche benutzt werden, wobei es über die Aufbewahrungstasche mit Energie versorgt wird. Figur 9 beschreibt ein weiteres Beispiel dieser Ausführungsform. Hierbei sind das Netzteil und ein Steckkontakt für die Steckdose in die Aufbewahrungstasche integriert.

## Patentansprüche

1. Ein System zur Bestimmung der Konzentration eines Analyten in einer Körperflüssigkeit beinhaltend
- ein medizinisches Instrument, das ein Auswertemodul zur Analyse von Daten für die Konzentrationsbestimmung aufweist,
- mindestens ein analytisches Testelement, das eine Nachweiszone mit Nachweischemie zum Nachweis des Analyten in einer Körperflüssigkeit aufweist, sowie
- eine Aufbewahrungstasche, in der das Instrument und das mindestens eine Testelement aufbewahrt werden,
wobei ein Einschaltmodul und/oder ein Modul zur Inventarkontrolle in der Aufbewahrungstasche integriert sind.

2. Ein System gemäß Anspruch 1, wobei das Einschaltmodul und/oder das Modul zur Inventarkontrolle fest in der Aufbewahrungstasche installiert sind.

3. Ein System gemäß Anspruch 1, wobei das Einschaltmodul und/oder das Modul zur Inventarkontrolle aus der Aufbewahrungstasche entnehmbar sind.

4. Ein System gemäß einem der Ansprüche 1 bis 3, wobei das System zusätzlich
- mindestens eine Lanzette zum Erzeugen einer Körperöffnung, sowie
- eine Stechhilfe, die die Bewegung der Lanzette bei der Erzeugung der Körperöffnung steuert, beinhaltet,
und in der Aufbewahrungstasche zusätzlich die mindestens eine Lanzette und die Stecheinheit aufbewahrt werden.

5. Ein System gemäß einem der Ansprüche 1 bis 4, wobei das System zusätzlich der Verabreichung eines Medikaments dient und
- ein Instrument, das ein Medikationsmodul zur Verabreichung eines Medikaments aufweist, beinhaltet.

6. Ein System gemäß einem der Ansprüche 1 bis 5, wobei die Kommunikation zwischen den ein oder mehreren in der Aufbewahrungstasche integrierten Modulen und anderen Modulen des Systems über elektrische Kontakte erfolgt.

7. Ein System gemäß einem der Ansprüche 1 bis 6, wobei die Kommunikation zwischen den ein oder mehreren in der Aufbewahrungstasche integrierten Modulen und anderen Modulen des Systems drahtlos erfolgt.

8. Eine Aufbewahrungstasche zur Aufbewahrung von Komponenten eines Systems zur Bestimmung der Konzentration eines Analyten in einer Körperflüssigkeit, in der
- ein medizinisches Instrument, das ein Auswertemodul zur Analyse von Daten für die Konzentrationsbestimmung aufweist, sowie
- mindestens ein analytisches Testelement, das eine Nachweiszone mit Nachweischemie zum Nachweis des Analyten in einer Körperflüssigkeit aufweist,
aufbewahrt werden,
wobei ein Einschaltmodul und/oder ein Modul zur Inventarkontrolle in der Aufbewahrungstasche integriert sind.

9. Eine Aufbewahrungstasche gemäß Anspruch 8, wobei das Einschaltmodul und/oder das Modul zur Inventarkontrolle fest in der Aufbewahrungstasche installiert sind.

10. Eine Aufbewahrungstasche gemäß Anspruch 8, wobei das Einschaltmodul und/oder das Modul zur Inventarkontrolle aus der Aufbewahrungstasche entnehmbar sind.
